# EUROPÄISCHE PATENTSCHRIFT

(11) **EP 1 022 989 B1**
(45) Veröffentlichungstag und Bekanntmachung des Hinweises auf die Patenterteilung: **29.12.2004**
(21) Anmeldenummer: 98961008.4
(22) Anmeldetag: 14.10.1998
(51) Int. Cl.: A61B 17/28, A61B 17/00

(54) **INSTRUMENT BZW. ZANGE FÜR MEDIZINISCHE UND INSBESONDERE ENDOSKOPISCHE ANWENDUNGEN**
INSTRUMENT OR FORCEPS FOR MEDICAL, IN PARTICULAR ENDOSCOPIC, USE
INSTRUMENT OU PINCE A USAGE MEDICAL ET NOTAMMENT ENDOSCOPIQUE

(30) Priorität: 14.10.1997 DE 19745157
(43) Veröffentlichungstag der Anmeldung: 02.08.2000
(73) Patentinhaber: Karl Storz GmbH & Co. KG, 78532 Tuttlingen (DE)
(72) Erfinder: CUSCHIERI, Alfred, Dundee DD1 9SY (GB); FRANK, Tim, Dundee DD1 9SY (GB)
(74) Vertreter: Lohr, Georg, Dr.
(86) Internationale Anmeldenummer: PCT/DE1998/003017
(87) Internationale Veröffentlichungsnummer: WO 1999/018863

(56) Entgegenhaltungen:
- EP-A- 0 640 319
- DE-A- 4 431 561
- US-A- 5 549 636
- US-A- 5 630 831

## Beschreibung

Die Erfindung bezieht sich auf ein Instrument für medizinische und insbesondere endoskopische Anwendungen gemäß dem Oberbegriff des Patentanspruchs 1.

### Stand der Technik

Es sind endoskopische Instrumente, wie beispielsweise Zangen bekannt, bei denen das Zangenmaul nicht nur eine einfache Öffnungs- bzw. Schließbewegung ausführen kann, wie dies bei der aus der WO 94/20034 bekannten Zange der Fall ist. Auf diese Druckschrift wird im übrigen zur Erläuterung aller hier nicht näher beschriebenen Einzelheiten ausdrücklich Bezug genommen.

So sind die verschiedensten Instrumente bekannt, die an ihrem distalen Ende zwei oder mehr Funktionselemente aufweisen, die geschwenkt oder in Richtung der Längsachse des Instruments verschoben werden können und dabei voneinander unabhängige Bewegungen sowie gegebenenfalls miteinander gekoppelte Bewegungen ausführen können.

Ein Beispiel für ein derartiges Instrument ist ein HF-Instrument mit einer verschiebbaren HF-Schlinge und gegebenenfalls einem zusätzlichen Element, wie einer verschiebbaren Messerklinge.

Weiterhin sind Instrumente bzw. Zangen bekannt, bei denen das Zangenmaul mehrteilig ausgebildet ist. Exemplarisch soll hierzu auf die DE 694 03 583 T2 verwiesen werden.

Ferner sind Manipulatoren mit mehrteiligen Funktionselementen beispielsweise zum Halten oder Spreizen von Gewebe u.a. aus der JP 06311984 A, der EP 0 688 538 A1 oder der US 5,549,636 bekannt.

Die Bedienung der Funktionselemente geschieht bei den bekannten Instrumenten in der Regel durch Scheren- oder Druckgriffe oder durch Elemente, in die die Bedienperson ihre Finger einlegen kann. Diese Elemente übersetzen die Fingerbewegung in eine Bewegung der distalen Funktionselemente. Hierzu wird zusätzlich auf die US 5,630,831 oder die EP 0 613 762 A1 verwiesen.

Ferner sind Handgriffe mit Bedienelementen für mehrere Funktionen bekannt.

Sowohl aus der DE-A-44 31 561 wie auch aus der EP-A-0 640 319 sind endoskopische Instrumente bekannt, welche eine proximal angeordnete Bedieneinheit aufweisen, die derart ausgebildet ist, dass sie mit einer Hand umgriffen werden kann, und die wenigstens zwei schwenkbaren/kippbare Elemente aufweist, die Bestandteil der Oberfläche der Bedieneinheit sind. Weiterhin ist ein distal angeordnetes mehrteiliges Funktionselement vorgesehen, dessen einzelne Teile gemeinsam und zusätzlich unabhängige bewegbar sind. Weiterhin ist jeweils ein Teil dieses distal angeordneten mehrteiligen Funktionselements durch die Bewegung von wenigstens zwei Elementen steuerbar. Ein längliches Verbindungsteil ist zwischen der Bedieneinheit und dem mehrteiligen Funktionselement angeordnet.

Die beim Stand der Technik verwendeten Bedieneinheiten sind jedoch gerade dann, wenn mehr als ein distales Funktionselement bedient werden soll, unter ergonomischen Gesichtspunkten unbefriedigend. Darüberhinaus sind die bekannten Bedieneinheiten nicht intuitiv. Die vom Bediener am Bedienelement ausgeführte Bewegung ist häufig der vom entsprechenden Funktionselement ausgeführten Bewegung in Richtung und Art der Bewegung wenig ähnlich.

Dies kann zu u.U. schwerwiegenden Fehlern bei der Bedienung dieser Instrumente während einer Operation führen.

### Darstellung der Erfindung

Der Erfindung liegt die Aufgabe zugrunde, die Bedienung von Instrumenten insbesondere mit mehreren Funktionselementen so zu vereinfachen, daß eine intuitive, einfache und sichere Bedienung möglich ist.

Eine erfindungsgemäße Lösungen dieser Aufgabe ist im Patentanspruch 1 angegeben. Weiterbildungen der Erfindung sind Gegenstand der abhängigen Ansprüche.

Erfindungsgemäß ist die Bedieneinheit derart ausgebildet ist, daß sie mit einer Hand umgriffen werden kann, und wenigstens zwei schwenkbare und/oder kippbare Elemente aufweist, die Bestandteil der Oberfläche der Bedieneinheit sind, und durch deren Bewegung jeweils ein Teil des mehrteiligen Funktionselements gesteuert wird. Die Elemente sind zumindest annähernd als Segmente eines Rotationskörpers ausgebildet. Besonders bevorzugt ist es, wenn die Bedieneinheit so ausgebildet ist, daß sie auch als Handgriff zum Halten des Instruments dient.

Die Bedienperson kann damit das Instrument mit einer einzigen Hand halten und gleichzeitig durch die Bewegung einzelner Finger bzw. Fingerglieder die distal angeordneten Funktionselemente steuern. Das Greifen wird weiter erleichtert, wenn die Elemente zumindest annähernd als Segmente eines Rotationskörpers, wie eines Zylinders oder einer Kugel ausgebildet sind.

In jedem Falle hat die erfindungsgemäße Ausbildung den besonderen Vorteil, daß die Bedienperson die Bedieneinheit mit der ganzen Hand umgreifen kann, wobei der hintere Teil am Handballen anliegt und damit fixiert wird. Dabei kann ein Druck des Handballens gegen das hintere Ende dazu dienen, daß die Funktionselemente als Einheit, d.h. gemeinsam bewegt werden.

Eine weitere ergonomische Verbesserung erhält man, wenn in den Elementen Griffmulden für die Finger vorgesehen sind. Hierdurch wird insbesondere die intuitive Bedienung unterstützt.

Unter ergonomischen Gesichtspunkten ist es besonders vorteilhaft, wenn zwei miteinander gekoppelte Elemente vorgesehen sind, die in unterschiedliche Richtungen zum Bewegen der einzelnen Teile der Funktionselemente kippbar sind. Die Elemente sind bevorzugt über Gestänge mit Gleitelementen verbunden, die wiederum mit Stangen verbunden sind, die die Funktionselemente am distalen Ende bewegen.

Bei einer alternativen Ausführungsform der Erfindung können vier Segmente vorgesehen sein, von denen jeweils zwei miteinander gekoppelt sind. Die miteinander gekoppelten Segmente sind symmetrisch zu einer Ebene ausgebildet, die die Längsachse des Verbindungsteils enthält.

Durch die miteinander gekoppelten Segmente, die symmetrisch zu einer Ebene ausgebildet sind, die die Längsachse des Verbindungsteils enthält, erhält man eine symmetrische und Kippmoment-freie Betätigung der am distalen Ende vorgesehenen Funktionselemente.

Beide Ausbildungen können zur Betätigung eines oder mehrerer Funktionselemente eingesetzt werden.

Dabei können die proximalseitig angeordneten Segmente an einem feststehenden Teil des Instruments angelenkt und über eine Schubstange mit einem Gleitelement gekoppelt sein, das mit der zweiten Übertragungsstange verbunden ist. Die distalseitig angeordneten Segmente sind dann an den proximalseitig angeordneten Segmenten angelenkt und über eine Schubstange mit einem Gleitelement gekoppelt, das mit der ersten Übertragungsstange verbunden ist.

Weiterhin ist es von Vorteil, wenn die Bedienelemente in eine Stellung vorgespannt sind, in der die Funktionselemente eine bestimmte Stellung haben. Im Falle von als Zangenmaul ausgebildeten Funktionenselementen ist es dann bevorzugt, wenn die bestimmte Stellung die Stellung ist, in der das Zangenmaul geöffnet ist.

Unabhängig von der speziellen Ausbildung des Instruments bzw. der Zange ist es auch unter dem folgenden Gesichtspunkt bevorzugt, daß die Bedienelemente in eine Stellung vorgespannt sind, in der die Funktions- bzw. Maulelemente eine bestimmte Stellung haben, da dann die das Instrument bedienende Person durch Loslassen der Bedieneinheit das Instrument immer in eine Ausgangs- bzw. Grundstellung überführen kann. Diese "Grundstellung" kann beispielsweise die Stellung sein, in der die beiden Teile jedes Maulelements geöffnet sind.

Darüberhinaus ist es von Vorteil, wenn Feststellelemente vorgesehen sind, mit denen die Teile jedes Funktions- bzw. Maulelements in einer bestimmten Stellung festlegbar sind. Damit kann die Bedienperson das Instrument in einer für den jeweiligen Operations- bzw. Untersuchungsvorgang vorteilhaften bzw. ergonomisch günstigen Stellung festlegen.

Weiterhin ist es von Vorteil, wenn das Verbindungsteil und damit das an seinem distalen Ende angeordnete Funktions- bzw. Maulelement um seine Längsachse drehbar ist, wie dies beispielsweise aus der WO 94/20034 bekannt ist. Zum Drehen des distalen Endes kann an dem Verbindungsteil ein Rad vorgesehen sein, dessen Betätigung das Verbindungsteil dreht. Hierbei können verschiedene Raststellungen vorgesehen sein.

Die erfindungsgemäßen Lösungen gelten sowohl für starre als auch für flexible Instrumente.

Sofern die mehrteiligen Funktionselemente mehrteilige Zangen sind, können diese nicht nur eine reine Zangenfunktion, sondern selbstverständlich auch eine Scheren- bzw. Schneidfunktion haben. Dabei können alle bekannten Maulformen verwendet werden; auch eine Aufrauhung der Oberfläche kann vorgesehen sein, um seitliches Ausweichen des Objekts zu verhindern.

Im Anspruch 17 ist als Beispiel für ein mehrteiliges Funktionselement eine mehrteilige Zange angegeben, bei der jedes bewegliche Maulelement aus zwei Teilen besteht, von denen ein Teil, nämlich der proximalseitige Teil, an dem Verbindungsteil und der andere Teil, nämlich der distalseitige Teil, an dem proximalen Teil direkt oder über einen Hebelmechanismus angelenkt ist. Mittels der Bedieneinheit sind die proximalseitigen Teile der beiden Maulelemente und die distalseitigen Teile der beiden Maulelemente unabhängig voneinander beweglich. Damit ist es möglich, die beiden Maulelemente nicht nur als Einheit schwenken zu können, sondern sie auch ähnlich wie die Glieder eines Fingers abzuwinkeln. Hierdurch ist es beispielsweise möglich, beim Greifen eines Gewebestranges diesen "umgreifend" und nicht nur einseitig "quetschend"zu halten. Empfindliche, auch größere Objekte können schonend und sicher gegriffen und werden. Dennoch kann das Zangenmaul auf einen sehr kleinen Durchmesser zusammengelegt werden.

Ausdrücklich soll jedoch darauf hingewiesen werden, daß selbstverständlich die verschiedensten Funktionselemente, die keineswegs nur Zangen bzw. Scheren sein müssen, sondern in beliebiger Weise - wie aus dem Stand der Technik bekannt - ausgebildet sein können, mit der erfindungsgemäß ausgebildeten Bedieneinheit gesteuert werden können.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen hinsichtlich der Offenbarung aller im Text nicht näher erläuterten erfindungsgemäßen Einzelheiten ausdrücklich verwiesen wird. Es zeigen:
- Fig. 1: ein erstes Ausführungsbeispiel für eine erfindungsgemäß ausgebildete Bedieneinheit in einer auseinander gezogenen Darstellung,
- Fig. 2: ein zweites Ausführungsbeispiel für eine erfindungsgemäß ausgebildete Bedieneinheit in einer perspektivischen Darstellung,
- Fig. 3a-3c: Schnittzeichnungen durch die Bedieneinheit zur Erläuterung der Funktionsweise, und
- Fig. 4a-4c: ein Beispiel perspektivisch den Innenaufbau des zweiten Ausführungsbeispiels.

### Darstellung von Ausführungsbeispielen

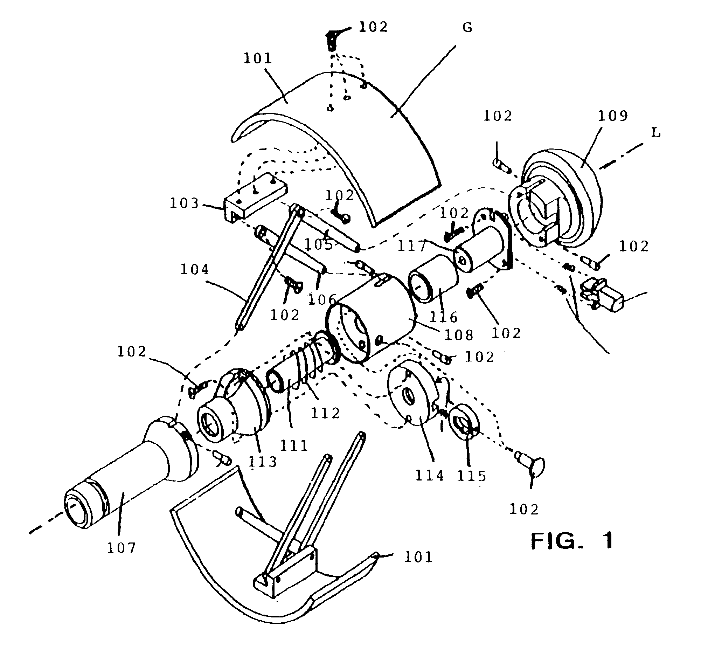

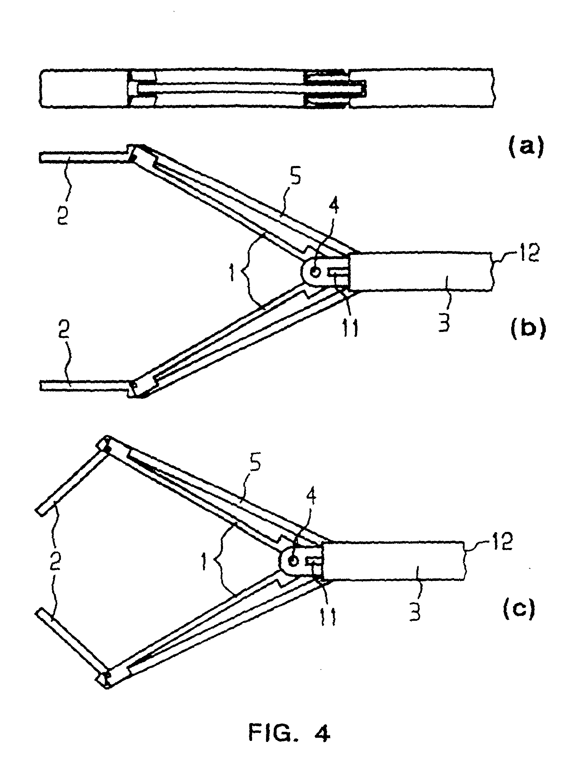

Fig. 1 zeigt in einer auseinandergezogenen Darstellung die Bedieneinheit eines ersten Ausführungsbeispiels eines erfindungsgemäßen Instruments. Der distale Bereich, der in beliebiger Weise ausgebildet sein kann und lediglich exemplarisch in Fig. 4 gezeigt ist, ist nicht dargestellt.

Die Bedieneinheit weist zwei in Richtung der Längsachse L des Instruments nach vorne und nach hinten kippbare Elemente 101 auf, die Bestandteil der Oberfläche der Bedieneinheit sind. An jedem Element 101 ist mittels Schrauben 102 ein Befestigungsblock 103 angebracht, an dem wiederum mittels Schrauben 102 ein Gestänge angelenkt ist, das aus drei Stangen 104, 105 und 106 besteht. Auf den Elementen 101 können ergonomisch angepaßte Griffmulden G, die nicht im einzelnen dargestellt sind, angebracht sein.

Die Stange 104 ist am hinteren bzw. proximalen (in Richtung der Längsachse L) Ende des Befestigungsblocks 103 zusammen mit der Stange 105 angelenkt, während die Stange 106 am vorderen Ende angelenkt ist. Die jeweils anderen Enden der Stangen 104, 105 und 106 sind an einem Gleitelement 107, einer Buchse 108 bzw. am hinteren Endteil 109 der Bedieneinheit angelenkt.

Am distalen Ende des Gleitelements 107 ist ein als Rohr ausgebildetes zweites Übertragungselement (in dieser Fig. nicht und lediglich in Fig. 4 dargestellt) befestigt, das die Bewegung des Gleitelements auf ein in Fig. 1 ebenfalls nicht dargestelltes distales Funktionselement überträgt. In dem Rohr ist koaxial ein erstes Übertragungselement angeordnet.

Mit dem Gleitelement 107 ist ein Zapfen 111, auf dem ein Feder 112 geführt ist, fest verbunden. Der Zapfen 111 ist in einer Führung 113 geführt, die über Schrauben 102 und Zwischenteile 114 und 115 an der Buchse 108 befestigt ist. An der Buchse 108 ist auch - wie bereits ausgeführt - das andere Ende der Stange 105 angelenkt. Am proximalen Ende der Buchse 108 ist eine Hülse 116 angeklebt, die auf einem Zapfen 117 geführt ist, der an dem proximalen Endteil 109 angeschraubt ist. Zu weiteren Einzelheiten wird auf die Zeichnung verwiesen.

Durch Kippen der Elemente 101 nach vorne bzw. nach hinten können die beiden Übertragungselemente einzeln nach vorne verschoben werden und damit die Funktionselemente einzeln betätigt werden. Durch Drücken des proximalen Endteils 109 nach vorne können die Funktionselemente als Einheit bewegt werden.

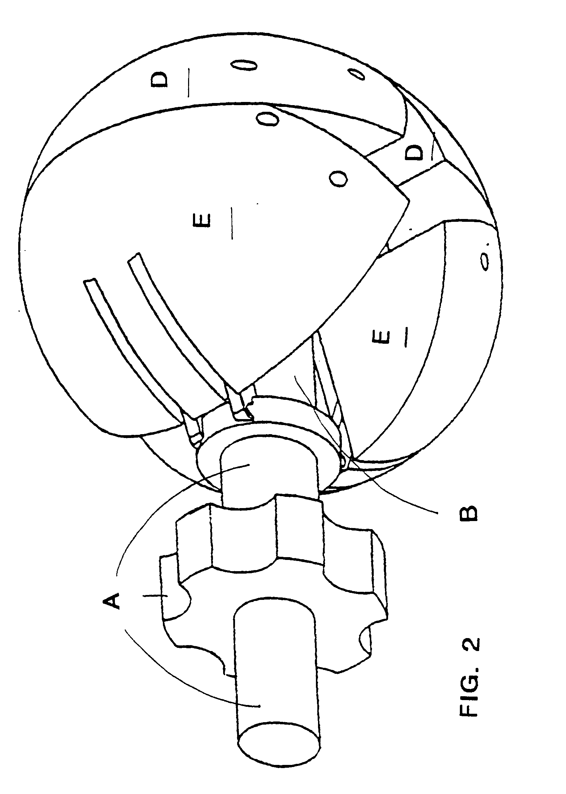

Fig. 2 zeigt perspektivisch die Bedieneinheit eines zweiten Ausführungsbeispiels, die anstelle der in Fig. 1 dargestellten Bedieneinheit eingesetzt werden kann. Darüberhinaus ist diese Bedieneinheit - in einer vereinfachten Ausführung - auch für Instrumente geeignet, bei denen lediglich ein distales Funktionselement durch eine Verschiebung einer Stange in dem Verbindungsteil betätigt wird.

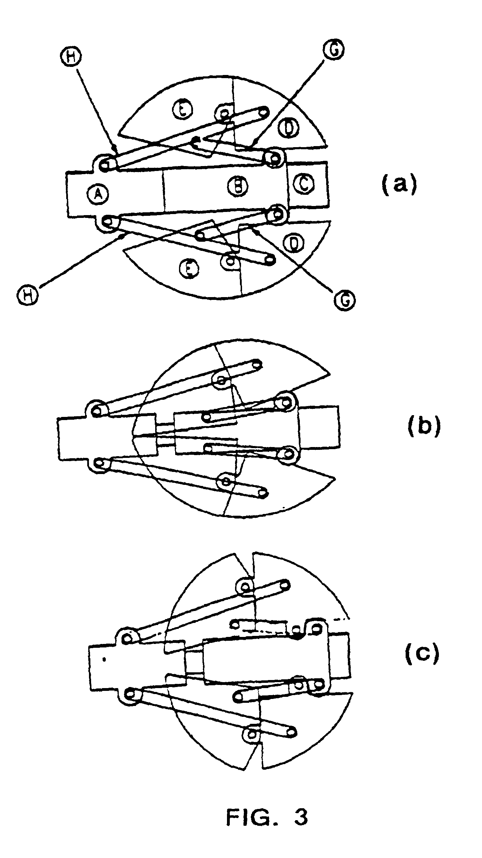

Die Funktionsweise des zweiten Ausführungsbeispiels wird durch die in den Fig. 3a-c dargestellten Schnittzeichnungen erläutert.

Auch bei diesem Ausführungsbeispiel ist die Bedieneinheit zugleich Handgriff. Die Bedieneinheit wird von der Bedienungsperson so in eine Hand genommen, daß sie von der Hand umschlossen wird und mit ihrem proximalen Ende am Handballen anliegt.

Bei dem gezeigten zweiten Ausführungsbeispiel hat die Bedieneinheit die Form einer Kugel, die in vier Segmente D, D, E und E unterteilt ist, von denen jeweils zwei, nämlich die Segmente D und D bzw. E und E miteinander gekoppelt sind. Die miteinander gekoppelten Segmente sind symmetrisch zu einer Ebene ausgebildet, die die Längsachse L des Verbindungsteils enthält.

Die proximalseitig angeordneten Segmente D sind an einem feststehenden Teil C des Instruments angelenkt und über eine Schubstange H mit einem Gleitelement A gekoppelt, das mit der zweiten Übertragungsstange verbunden ist. Die distalseitig angeordneten Segmente E sind an den proximalseitig angeordneten Segmenten D angelenkt und über eine Schubstange G mit einem Gleitelement B gekoppelt, das mit der ersten Übertragungsstange verbunden ist. Die Elemente A und C gleiten auf dem Element B, während sich das Element E auf D und D auf B dreht.

Die Figuren 3a bis 3c zeigen unterschiedliche Stellungen des Bedienelements, wobei gleiche Teile mit den selben Bezugszeichen versehen sind.

Fig. 3a zeigt die Grundstellung, in die die einzelnen Segmente, d.h. die einzelnen Bedienelemente beispielsweise durch Federn vorgespannt sein können.

Fig. 3b zeigt die Stellung, in der lediglich die Segmente D betätigt sind. Hierdurch wird das Gleitelement A, das mit der zweiten Übertragungsstange verbunden ist, verschoben, so daß (beispielsweise im Falle einer zweiteiligen Zange) die distalseitigen Teile des Maulelements betätigt werden.

Fig. 3c zeigt die Stellung, in der die vorderen Segmente E betätigt sind. Hierdurch wird das Gleitelement B, das mit der ersten Übertragungsstange verbunden ist, verschoben, so daß beim vorigen Beispiel die proximalseitigen Teile des Maulelements betätigt werden.

In den Figuren 4a-4c ist ein Zangenmaul als Beispiel für ein mögliches Funktionselement in verschiedenen Stellungen, nämlich in der geschlossenen (Fig. 4a), der vollständig geöffneten Stellung (Fig. 4b) und in einer Stellung gezeigt, in der beispielsweise ein Gewebestrang umgriffen werden kann (Fig. 4c).

Das dargestellte Zangenmaul weist in bekannter Weise zwei Maulelemente auf. Jedes Maulelement besteht aus zwei Teilen, nämlich einem proximalseitigem Teil 1, der an einem Verbindungsteil 3 zu einem proximalen Teil der Zange angelenkt ist, und einem distalseitigen Teil 2, der an dem proximalseitigen Teil 1 des Maulelements angelenkt ist.

In dem Verbindungsteil 3 sind zwei Übertragungsstangen 11 und 12 vorgesehen, von denen die Stange 12 als Rohr ausgeführt ist und die Stange 11 umgibt. Eine Verschiebung der Stange 11 verschwenkt ein Element 4, dessen Bewegung die Teile 1 öffnet bzw. schließt. Eine Verschiebung der Stange 12 bewirkt über ein Gestänge 5 eine Schwenkbewegung der Teile 2 relativ zu den Teilen 1, so daß die distalseitig angeordneten Teile 2 des Zangenmauls unabhängig von den proximalseitig angeordneten Teilen 1 bewegt werden können. Dies zeigen die Figuren 4a bis 4c.

Vorstehend ist die Erfindung ohne Beschränkung des allgemeinen Erfindungsgedankens und insbesondere der Verwendung beliebiger distaler Funktionselemente beschrieben worden.

## Patentansprüche

1. Instrument für medizinische und insbesondere endoskopische Anwendungen, mit
- einem distal angeordneten mehrteiligen Funktionselement (1,2,5) wie einem mehrteiligen Zangenmaul oder einer Schere, dessen einzelne Teile gemeinsam und zusätzlich unabhängig bewegbar sind,
- einer proximal angeordneten Bedieneinheit, umfassend ein ergonomisch geformtes Griffteil (D,D,E,E), wobei die Bedieneinheit derart ausgebildet ist, dass sie mit einer Hand umgriffen werden kann, und wenigstens zwei schwenkbare und/oder kippbare Elemente (101) aufweist, wobei durch die Bewegung der schwenkbaren und/oder kippbaren Elemente (101) jeweils ein Teil des mehrteiligen Funktionselements gesteuert wird,
- einem länglichen Verbindungsteil (3), das zwischen Bedieneinheit und Funktionselement angeordnet ist,
**dadurch gekennzeichnet, dass** die Elemente Bestandteile der Oberfläche der Bedieneinheit sind und zumindest annähernd als Segmente eines Rotationskörpers ausgebildet sind.

2. Instrument nach Anspruch 1,
**dadurch gekennzeichnet, dass** die Bedieneinheit gleichzeitig als Handgriff zum Halten des Instruments ausgebildet ist.

3. Instrument nach einem der Ansprüche 1 bis 2,
**dadurch gekennzeichnet, daß** das hintere Ende der Bedieneinheit mit einem mehrteiligen Gestänge (104, 105, 106) verbunden ist, das in einem ebenfalls mehrteiligen Befestigungsblock (103) geführt wird und die Funktionselemente mit dem Gestänge (104, 105, 106) derart verbunden sind, dass sie als Einheit durch die Bewegung des hinteren Ende des Bedienteiles verschoben werden.

4. Instrument nach einem der Ansprüche 1 bis 3,
**dadurch gekennzeichnet, daß** der Rotationskörper, als Zylinder oder Kugel ausgebildet ist.

5. Instrument nach einem der Ansprüche 1 bis 4,
**dadurch gekennzeichnet, daß** in den Elementen Griffmulden für die Finger vorgesehen sind.

6. Instrument nach einem der Ansprüche 1 bis 5,
**dadurch gekennzeichnet, daß** zwei Elemente vorgesehen sind, die in unterschiedliche Richtungen zum Bewegen der einzelnen Teile der Funktionselemente kippbar sind.

7. Instrument nach Anspruch 6,
**dadurch gekennzeichnet, daß** die Elemente über Gestänge (104, 105,106) mit Gleitelementen (107) verbunden sind, die wiederum mit Stangen (104, 105, 106) verbunden sind, die die Funktionselemente bewegen.

8. Instrument nach einem der Ansprüche 1 bis 4,
**dadurch gekennzeichnet, daß** vier Segmente (D, D, E, E) vorgesehen sind, von denen jeweils zwei miteinander gekoppelt sind.

9. Instrument nach Anspruch 8,
**dadurch gekennzeichnet, daß** die miteinander gekoppelten Segmente (D, D, E, E) symmetrisch zu einer Ebene ausgebildet sind, die die Längsachse (L) des Verbindungsteils enthält.

10. Instrument nach Anspruch 8 oder 9,
**dadurch gekennzeichnet, daß** die proximalseitig angeordneten Segmente (D, E,) an einem feststehenden Teil (C) des Instruments angelenkt und über eine Schubstange (H) mit einem Gleitelement (A, B) gekoppelt sind, das mit einer zweiten Schubstange (G) verbunden ist, und
daß die distalseitig angeordneten Segmente (D, E) an den proximalseitig angeordneten Segmenten (D, E) angelenkt und über eine Schubstange (G) mit einem Gleitelement (A, B) gekoppelt sind, das mit der ersten Schubstange (H) verbunden ist.

11. Instrument nach einem der Ansprüche 1 bis 9,
**dadurch gekennzeichnet, daß** die Bedienelemente in eine Stellung vorspannbar sind, in der die Funktionselemente eine bestimmte Stellung haben.

12. Instrument nach Anspruch 11,
**dadurch gekennzeichnet, daß** die bestimmte Stellung die Stellung ist, in der die Teile eines als Zangenmaul ausgebildeten Funktionselements geöffnet sind.

13. Instrument nach einem der Ansprüche 1 bis 11,
**dadurch gekennzeichnet, daß** Feststellelemente vorgesehen sind, mit denen die Teile jedes Funktionselements in einer bestimmten Stellung festlegbar sind.

14. Instrument nach einem der Ansprüche 1 bis 12,
**dadurch gekennzeichnet, daß** das Verbindungsteil und damit das an seinem distalen Ende angeordnete Funktionselement um seine Längsachse drehbar ist.

15. Instrument nach Anspruch 14,
**dadurch gekennzeichnet, daß** an dem Verbindungsteil ein Rad vorgesehen ist, dessen Betätigung das Verbindungsteil dreht.

16. Instrument nach einem der Ansprüche 1 bis 15,
**dadurch gekennzeichnet, daß** die Funktionselemente Bestandteile eines mehrteiligen Zangenmauls sind.

17. Instrument nach Anspruch 16,
**gekennzeichnet durch** die Kombination folgender Merkmale:
- jedes Maulelement besteht aus wenigstens zwei Teilen, von denen ein proximalseitiges Teil (1) an dem Verbindungsteil (3) und ein anderes, distalseitiges Teil (2) an dem proximalseitigen Teil (1) angelenkt ist,
- mittels der Bedieneinheit ist das proximalseitige Teil (1) des wenigstens einen beweglichen Maulelementes relativ zum Verbindungsteil (3) und das distalseitige Teil (2) unabhängig vom proximalseitigen Teil (1) beweglich.

18. Instrument nach Anspruch 17,
**dadurch gekennzeichnet, daß** die proximal- und distalseitigen Teile (1, 2) schwenkbar sind.

19. Instrument nach Anspruch 16 oder 17,
**dadurch gekennzeichnet, daß** das Zangenmaul eine Greif- oder Klemmfunktion aufweist.

20. Instrument nach Anspruch 16 oder 17,
**dadurch gekennzeichnet, daß** das Zangenmaul eine Schneidund/oder Entnahmefunktion aufweist.

21. Instrument nach Anspruch 16 oder 17,
**dadurch gekennzeichnet, daß** das Zangenmaul derart ausgebildet ist, daß es das Aufdrücken von Öffnungen erlaubt.

22. Instrument nach einem der Ansprüche 1 bis 20,
**dadurch gekennzeichnet, daß** die proximalseitigen Teile (1) der Maulelemente durch ein erstes Übertragungselement (11) mit dem zugeordneten Bedienelement der Bedieneinheit und die distalseitigen Teile (2) der Maulelemente durch ein Gestänge (5) und ein zweites Übertragungselement (12)mit dem zugeordneten Bedienelement der Bedieneinheit verbunden sind.

23. Instrument nach Anspruch 22,
**dadurch gekennzeichnet, daß** das erste Übertragungselement (11) koaxial in dem als Rohr ausgebildeten zweiten Übertragungselement (12) angeordnet ist.

## Claims

1. Instrument for medical and, in particular, endoscopic applications, comprising
- a distally disposed multipart functional element (1, 2, 5) such as multipart forceps jaws or cutters, the single parts of which are adapted to be moved jointly and also independently;
- a proximately disposed operating unit comprising an ergonomically shaped handle portion (D, D, E, E), the operating unit being designed in such manner that it may be gripped with one hand, and has at least two swivelling and/or tilting elements (101), wherein a movement of the swivelling and/or tilting elements (101) controls a respective part of the multipart functional element;
- an elongate connecting portion (3) disposed between the operating unit and the functional element;
**characterized in that** the elements are components of the surface of the operating unit and are designed at least approximately as segments of a solid of revolution.

2. Instrument according to claim 1,
**characterized in that** the operating unit is designed to be also a handle for holding the instrument.

3. Instrument according to any one of claims 1 to 2,
**characterized in that** the rear end of the operating unit is connected to a multipart rod linkage (104, 105 106) guided in an also multipart mounting block (103), and the functional elements are connected to the rod linkage (104, 105, 106) in such manner that they are caused to slide as a unit by a movement of the rear end of the operating portion.

4. Instrument according to any one of claims 1 to 3,
**characterized in that** the solid of revolution is designed to be a cylinder or a sphere.

5. Instrument according to any one of claims 1 to 4,
**characterized in that** grip-grooves for fingers are provided on the elements.

6. Instrument according to any one of claims 1 to 5,
**characterized in that** two elements are provided that can be tilted in different directions to move the individual parts of the functional elements.

7. Instrument according to claim 6,
**characterized in that** the elements are connected via rod linkages (104, 105, 106) to sliding elements (107) that in tum are connected to rods (104, 105, 106) which move the functional elements.

8. Instrument according to any one of claims 1 to 4,
**characterized in that** four segments (D, D, E, E) are provided, of which two at a time are coupled with each other.

9. Instrument according to claim 8,
**characterized in that** the segments (D, D, E, E) coupled with each other are configured to be symmetrical to a plane containing the longitudinal axis (L) of the connecting portion.

10. Instrument according to claim 8 or 9,
**characterized in that** the segments (D, E) disposed on the proximal side are pivotally supported on a fixed part (C) of the instrument, and are coupled via a push-rod (H) with a sliding element (A, B) connected to a second push-rod (G); and
that the segments (D, E) disposed on the distal side are pivotally supported on the segments (D, E) disposed on the proximal side, and are coupled via a push-rod (G) with a sliding element (A,B) connected to the first push-rod (H).

11. Instrument according to any one of claims 1 to 9,
**characterized in that** the operating elements are biased into a position in which the functional elements are in a certain position.

12. Instrument according to claim 11,
**characterized in that** the certain position is the position in which the parts of a functional element designed to be forceps jaws are open.

13. Instrument according to any one of claims 1 to 11,
**characterized in that** locking elements are provided for locking the parts of each functional element in a certain position.

14. Instrument according to any one of claims 1 to 12,
**characterized in that** the connecting portion, and with it the functional element disposed at the distal end thereof, is rotatable about its longitudinal axis.

15. Instrument according to claim 14,
**characterized in that** a wheel is provided on the connecting portion, and actuation of the wheel rotates the connecting portion.

16. Instrument according to any one of claims 1 to 15,
**characterized in that** the functional elements are components of multipart forceps jaws.

17. Instrument according to claim 16,
**characterized by** the combination of the following features:
- each jaws element consists of at least two parts, of which a proximal side part (1) is pivotally supported on the connecting portion (3), and another, distal side part (2) is pivotally supported on the proximal side part (1);
- by means of the operating unit, the proximal side part (1) of the at least one movable jaws element can be moved relative to the connecting portion (3), and the distal side part (2) can be moved independently from the proximal side part (1).

18. Instrument according to claim 17,
**characterized in that** the proximal and distal side parts (1, 2) are adapted to swivel.

19. Instrument according to claim 16 or 17,
**characterized in that** the forceps jaws have a grasping or clamping function.

20. Instrument according to claim 16 or 17,
**characterized in that** the forceps jaws have a cutting and/or extracting function.

21. Instrument according to claim 16 or 17,
**characterized in that** the forceps jaws are configured to permit apertures to be urged open.

22. Instrument according to any one of claims 1 to 20,
**characterized in that** the proximal side parts (1) of the jaws elements are connected via a first transmission element (11) to the associated operating element of the operating unit, and the distal side parts (2) of the jaws elements are connected via a rod linkage (5) and a second transmission element (12) to the associated operating element of the operating unit.

23. Instrument according to claim 22,
**characterized in that** the first transmission element (11) is disposed coaxially within the second transmission element (12) that is configured as a tube.

## Revendications

1. Instrument à usage médical et notamment endoscopique, comprenant
- un élément fonctionnel (1, 2, 5) à plusieurs parties, disposé à une extrémité distale, par exemple une ouverture d'une pince ou d'une paire de ciseaux, chacune à plusieurs parties, dont les parties individuelles sont mobiles soit en commun, soit, au plus, indépendamment,
- une unité de commande, disposée à une extrémité proximale, comprenant une pièce de poignée à forme ergonomique (D, D, E, E), à l'unité de commande étant configurée d'une telle manière, qu'on puisse la saisir par un seul main, et comprenant au moins deux éléments (101) pivotables et/ou basculants, au mouvement desdits éléments (101) pivotables et/ou basculants commandant une partie respective dudit élément fonctionnel à plusieurs parties,
- une partie allongée de connexion (3), qui es disposée entre ladite unité de commande et ledit élément fonctionnel,
**caractérisé en ce que** lesdits éléments sont des composants de la surface de ladite unité de commande et sont configurés, au moins de façon approximative, sous forme de segments d'un corps de révolution.

2. Instrument selon la revendication 1,
**caractérisé en ce que** ladite unité de commande est configurée, en même temps, sous forme d'une poignée à tenir l'instrument.

3. Instrument selon une quelconque des revendications 1 à 2,
**caractérisé en ce que** l'extrémité arrière de ladite unité de commande est reliée à une timonerie (104, 105, 106) à plusieurs parties, qui est guidée dans un bloc fixateur (103), également à plusieurs parties, et **en ce que** lesdits éléments fonctionnels sont reliés à ladite timonerie (104, 105, 106) d'une telle manière, qu'ils soient déplacées en tant qu'une seule unité par le mouvement de l'extrémité arrière dudit élément de commande.

4. Instrument selon une quelconque des revendications 1 à 3,
**caractérisé en ce que** ledit corps de révolution est configuré sous forme d'un cylindre ou d'une sphère.

5. Instrument selon une quelconque des revendications 1 à 4,
**caractérisé en ce que** des poignées encastrées sont formées pour les doigts dans lesdits éléments.

6. Instrument selon une quelconque des revendications 1 à 5,
**caractérisé en ce que** deux éléments sont formés, qui sont basculants en directions différentes afin de mouvoir les parties individuelles desdits éléments fonctionnels.

7. Instrument selon la revendication 6,
**caractérisé en ce que** lesdits éléments sont reliés, via des tiges (104, 105, 106), aux éléments glissants (107), qui, à leur tour, sont reliées aux tiges (104, 105, 106) causant le mouvement desdits éléments fonctionnels.

8. Instrument selon une quelconque des revendications 1 à 4,
**caractérisé en ce que** quatre segments (D, D, E, E) sont prévus, dont deux sont accouplés l'un à l'autre en paires respectives.

9. Instrument selon la revendication 8,
**caractérisé en ce que** lesdits segments accouplés l'un à l'autre (D, D, E, E) sont formés en symétrie relative à un plan contenant l'axe longitudinale (L) dudit élément de connexion.

10. Instrument selon la revendication 8 ou 9,
**caractérisé en ce que** les segments (D, E) disposés du côté proximal sont articulés à une partie (c) stationnaire de l'instrument et accouplés à un élément glissant (A, B) via une bielle de commande (H), cet élément étant relié à une deuxième bielle de commande (G),
et **en ce que** les segments (D, E) disposés du côté distal sont articulés aux segments (D, E) disposés du côté proximal, en étant accouplé, via une bielle de commande (G), à un élément glissant (A, B) raccordé à ladite première bielle de commande (H).

11. Instrument selon une quelconque des revendications 1 à 9,
**caractérisé en ce que** lesdits éléments de commande sont aptes à être mis en précontrainte en une position, dans laquelle les éléments fonctionnels prennent une position définie.

12. Instrument selon la revendication 11,
**caractérisé en ce que** ladite position définie est la position, dans laquelle les parties d'un élément fonctionnel configuré sous forme d'une ouverture de pince sont ouvertes.

13. Instrument selon une quelconque des revendications 1 à 11,
**caractérisé en ce que** des éléments de blocage sont disposés, qui servent à arrêter les parties de chaque élément fonctionnel en une position définie.

14. Instrument selon une quelconque des revendications 1 à 12,
**caractérisé en ce que** la partie de connexion et ainsi l'élément fonctionnel disposé à son extrémité distale est rotative autour de son axe longitudinal.

15. Instrument selon la revendication 14,
**caractérisé en ce qu'**une roue est prévue à ladite partie de connexion, qui est commandée afin de tourner ladite partie de connexion.

16. Instrument selon une quelconque des revendications 1 à 15,
**caractérisé en ce que** lesdits éléments fonctionnels constituent des composants d'une ouverture de pince à plusieurs parties.

17. Instrument selon la revendication 16,
**caractérisé par** la combinaison des caractéristiques suivantes :
- chaque élément de mâchoire est constitué par au moins deux parties, dont une partie (1) du côté proximal est articulée à ladite partie de connexion (3), pendant qu'une autre partie (2) du côté distal est articulée à ladite partie (1) du côté proximal,
- ladite partie (1) du côté proximal dudit au moins un élément de mâchoire est mobile, moyennant de ladite unité de commande, relativement à ladite partie de connexion (3), pendant que la partie (2) du côté distal est mobile indépendamment de ladite partie (1) du côté proximal.

18. Instrument selon la revendication 17,
**caractérisé en ce que** lesdites parties (1, 2) du côté proximal et du côté distal sont pivotables.

19. Instrument selon la revendication 16 ou 17,
**caractérisé en ce que** ledit ouverture de pince présente une fonction de saisie ou de serrage.

20. Instrument selon la revendication 16 ou 17,
**caractérisé en ce que** ledit ouverture de pince présente une fonction de coupage et/ou de prélèvement.

21. Instrument selon la revendication 16 ou 17,
**caractérisé en ce que** ledit ouverture de pince a une telle configuration, qu'il permet l'écartement à pression des ouvertures.

22. Instrument selon une quelconque des revendications 1 à 20,
**caractérisé en ce que** lesdites parties (1) du côté proximal desdits éléments de mâchoire sont reliées, par un premier élément de transmission (11), à l'élément de commande y affecté de ladite unité de commande, pendant que lesdites parties (2) du côté distal desdits éléments de mâchoire sont reliées par une timonerie (5) et par un deuxième élément de transmission (12) à l'élément de commande y affecté de ladite unité de commande.

23. Instrument selon la revendication 22,
**caractérisé en ce que** ledit premier élément de transmission (11) est disposé en position coaxiale dans ledit deuxième élément de transmission (12) configuré sous forme d'un tube.
